# EUROPEAN PATENT APPLICATION

(11) **EP 1 917 972 A1**
(43) Date of publication of application: **07.05.2008**
(21) Application number: 06767446.5
(22) Date of filing: 27.06.2006
(51) Int. Cl.: A61K 38/16, A23L 1/305, A61K 8/64, A61P 1/16, A61K 39/02, A61P 39/04

(54) **AGENT FOR AMELIORATING HEAVY METAL-INDUCED DISORDERS, AND MEDICINAL COMPOSITION, FOOD AND COSMETIC CONTAINING THE SAME**

(30) Priority: 29.06.2005 JP 2005189619
(71) Applicant: NRL Pharma, Inc., Kawasaki-shi, Kanagawa 2130012 (JP)
(72) Inventor: TSUBOTA, Akihito, Yokohama-shi, Kanagawa 224-0029 (JP)
(74) Representative: Brochard, Pascale
(86) International application number: PCT/JP2006/312829
(87) International publication number: WO 2007/001006

(57) **Abstract**

It is intended to provide a drug for ameliorating symptoms or diseases caused by heavy metals (for example, Wilson's disease, heavy metal toxication, aging, fulminant hepatitis and so on) which has a high safety without any fear of side effect, can eliminate heavy metals such as copper ion accumulated in excess in the living body to prevent or lessen the accumulation of the heavy metals in the living body, thereby eliminating or relieving the effects of the heavy metals; and compositions such as a medicinal composition, a food and a cosmetic containing the same. The drug and compositions as described above are **characterized by** containing lactoferrin and/or an active derivative of the same as the active ingredient.

## Description

### TECHNICAL FIELD

The present invention relates to an agent containing lactoferrin as an active ingredient for ameliorating symptoms or diseases caused by heavy metals, such as Wilson's disease, heavy metal intoxication and aging, and a medicinal composition, a food and a cosmetic containing the same.

### BACKGROUND ART

Wilson's disease is a congenital disorder of copper metabolism, and by copper abnormally accumulated in the body, it develops a severe progressive hepatic disorder and a central nerve disorder in early childhood [Nonpatent Document 11. The cause of the disease is the genetic abnormality of a membrane protein (transporter) called ATP7B, which is in charge for the intracellular transport of copper [Nonpatent Document 2]. The incidence of Wilson's disease is reported to be 2 to 3 patients per 100,000 people.

Wilson's disease is a lethal congenital disorder in the past. Because the pathology of the disease has been clarified over a century, the treatment has been made possible before symptoms of a hepatic disorder and a central nerve disorder is exhibited, by promoting the excretion of copper using a chelating agent which traps accumulating copper.

Presently, the diagnosis and treatment of Wilson's disease are almost established. However, because the treatment is premised on an early detection, the development and introduction of methods for early diagnosis are strongly desired. Further, the present treatment involves many problems.

In Japan, two kinds of copper chelating agents, D-penicillamine [Nonpatent Document 3] and trientine hydrochloride [Nonpatent Document 4] are used as the therapeutics.

Penicillamine was originally developed as a therapeutic agent for treating rheumatoid arthritis, because it has actions for delaying the progression of rheumatoid arthritis, improving abnormal immunity and mitigating articular inflammation. Penicillamine has recently become used for the treatment of Wilson's disease and poisoning symptoms caused by lead, mercury and copper, because it forms strong complex compounds with heavy-metal ions. Adult patients of Wilson's disease are usually orally administered 1,000 mg per day of penicillamine in one to several divided doses in the fasting state before meal. Penicillamine needs to be administered in combination with vitamin B₆ because it is antagonistic to vitamin B₆. Further, due to its strong immunosuppressive action and inhibitory action on collagen synthesis, penicillamine has become rarely used for treating rheumatoid arthritis. Moreover, penicillamine may cause a severe blood disorder such as agranulocytosis. Thus, it should be carefully used. Side effects which often occur include exanthema, itch of the skin, nausea, stomatitis, numbness in limbs, abnormal gestation and the like. Severe side effects for which particular attention should be paid are a blood disorder, a kidney disorder and interstitial pneumonia, and decreased blood cells, urinary proteins and hematuria may occur. Although these side effects rarely become serious, the initial symptoms such as fever, sore throat, bleeding tendency, dry cough and closeness should be carefully watched. In addition, new diseases of the immune system, such as myasthenia, polymyositis and systemic lupus erythematosus may occur, though the possibility is low.

Trientine hydrochloride (N,N'-Bis(2-aminoethyl)- 1,2-ethanediamine hydrochloride salt) is a copper chelating agent developed as an orphan drug by Merck & Co., Inc., U. S. A. [Nonpatent Document 5]. Trientine forms a complex compound with a copper ion at a ratio of 1:1, resulting in the excretion of the copper into urine. The indication of trientine hydrochloride is for D-penicillamine intolerance patients. Since many reports have shown its significant effects on Wilson's disease's patients who have nervous symptoms, trientine hydrochloride is requested to be listed as a first-line drug of Wilson's disease together with D-penicillamine. However, compared with penicillamine, trientine hydrochloride has disadvantages of its weak chelating action and the lack of experiences of long-term administration.

Zinc sulfate, zinc acetate [Nonpatent Document 6] and zinc gluconate have also been used as therapeutic agents for Wilson's disease. In 1997, U. S. FDA (the Food and Drug Administration) approved zinc acetate as a therapeutic agent for Wilson's disease. The pharmacological action of zinc is to inhibit competitively absorption of copper included in foods. Therefore, zinc does not have an effect on patients in the acute phase or the exacerbation phase of Wilson's disease, but it is considered to be effective for patients in the maintenance phase of treatment, affected infants before the onset of symptoms and patients in pregnancy, when administered alone for a long term or in combination with a copper chelating agent. It is reported that about 10 % of the patients complain abdominal discomfort, but zinc has a high level of safety with no severe side effects reported. Moreover, it is reported that zinc formulations did not have an impact on newborn babies in cases where they had been continuously administered to their mothers in pregnancy.

Walshe reported that tetrathiomolybdate (TTM) could become a new therapeutic drug for Wilson's disease [Nonpatent Document 7]. In European countries and the U.S., TTM has been clinically applied as the third copper chelating agent, and is considered to be effective particularly for cases with nervous symptoms. The effect of TTM in excreting copper is potent. TTM is incorporated into liver cells and actively excretes copper ions. The pathway of excretion is said to be related to the gastrointestinal tract, but the details should be revealed in the future. TTM is administered not only orally, but also transvenously. It is said that, when orally administered, TTM forms a strong chelate bond with the copper included in foods, and has another effect of inhibiting the absorption of copper from the intestinal tract.

The above-mentioned treatments require patients to take a copper chelating agent which frequently cause side effects through their lives. Moreover, copper chelating agents have a problem of accumulating iron in exchange for excreting copper. Although iron is essential for important reactions of biological components including hemoglobin, it is, on the other hand, a catalyst which accelerates the chain reaction of peroxide formation in vivo. Therefore, it is supposed that an excessive amount of iron not only promotes aging but also involves in the exacerbation of lifestyle-related diseases.

Moreover, Wilson's disease is accompanied by hypoceruloplasminemia before starting a treatment, and the treatment with a chelating agent further decreases available copper [Nonpatent Document 8]. More specifically, patients with Wilson's disease have lowered activity of serum ceruloplasmin, which is a ferroxidase involved in the iron transportation. Because holoceruloplasmin, which is bound to copper and has high ferroxidase activity, is particularly decreased, iron transportation deteriorates and iron deposition is easy to occur.

Therefore, as to Wilson's disease, the accumulation of iron and the treatment with copper-chelating agents are two sides of the same coin. In case the iron accumulation is prolonged or exacerbated, it is necessary to remove iron by bloodletting [Nonpatent Document 9]. At present, in order to treat the excessive iron accumulation, we have no appropriate therapy other than to remove the iron as hemoglobin from the body by bloodletting.

Lactoferrin (hereinafter also referred to as "LF") is a glycoprotein included in mammal's milk and forms a complex compound with water soluble iron. Its molecular weight is 86,000 in cows and 88,000 in humans. It is considered that lactoferrin, by depriving solutions of iron ions, show many kinds of physiological actions [Nonpatent Document 10].

It has been considered that LF is one of the defense factors which mothers give their babies whose acquired immunity is immature. Humans (Homo sapiens) are born with the immune system and nervous system which are immature, and are considered to be largely dependent on lactoferrin. Because newborn babies take large amounts of LF from breast milk, it is speculated that LF has effects on maturation of the immune system and nervous system. LF still exists after weaning, for example, in granules of mature neutrophils, the mucus of exocrine glands and so on. LF forms a strong complex compound with a trivalent iron ion (ferric ion), and shows a protective action to infections by removing ferric ions from the environment. Further, LF promotes the production of interleukin-18 by immune cells, and plays a role in suppressing inflammation by bacterial infections and protecting the body.

That LF has a high level of safety has been shown in the Provisional Publication [Patent Document 1] (Japanese Provisional Patent Publication (Laid-Open) No. 2002-161050, "New Pharmaceutical Composition for Ameliorating Quality of Life, Method for Producing New Food and Use Thereof")], which was filed by one of the present inventors. For example, LF showed no toxicity when it was administered to male and female beagle dogs and rats at single oral dose of 5 g/kg body weight, or at consecutive oral doses of 2 g/kg for 12 weeks. In fact, even though LF is added to modified milk powder for infants, health foods, yogurts and drinks, and numerous persons ranging from infants to aged persons have been taking them for a long time, there has been no report so far which calls the safety of LF into question.

In 1987, E. N. Baker et al. clarified the structure of LF by X-ray diffraction of carbonate crystal of human holo-lactoferrin which chelated a ferric ion, prepared from breast milk lactoferrin [Nonpatent Document 11]. LF consists of two roughly equal bulbous portions connected through a peptide chain which corresponds to a hinge, and thus the carboxyl terminal and amino terminal portions were designated as the C-lobe and the N-lobe, respectively. Inside of each of the hollow lobes, ferric ions are bound in the lobes by an electrostatic bond such as ion bond, hydrogen ionic bond and ion dipole, and form a complex compound. Human lactoferrin has a very high affinity to ferric ions, and its binding constant is 10²²M [Nonpatent Document 12]. Therefore, free ferric ions can not exist at a concentration of more than 10⁻¹⁸M in the presence of lactoferrin.

Following research on the structure of lactoferrin has revealed that, in spite of the differences of animal species, LF shows surprising structural homologies. At present, the structures of bovine lactoferrin obtained from cow milk [Nonpatent Document 13], lactoferrin of buffalo milk [Nonpatent Document 14] and lactoferrin of horse milk [Nonpatent Document 15] are determined to have the iron saturated holo-form by X-ray diffraction, and their three-dimensional structures are established as the same as that of human holo-lactoferrin. Interestingly, it is known that lactoferrin forms a strong complex compound with copper ion, and that the structure of the complex compound is almost the same as that of the one formed with iron [Nonpatent Document 16].

On the other hand, Purina et al. reported that human lactoferrin forms a complex with ceruloplasmin in vitro and in vivo [Nonpatent Document 17]. This complex dissociates by the presence of a high concentration of salt, calcium chloride or EDTA, or by lowering pH to 4.7. Moreover, it is known that DNA, bacterial lipopolysaccharide (LPS) and heparin etc. dissociates ceruloplasmin from a lactoferrin-ceruloplasmin complex by binding with lactoferrin. It is unclear why the complex of both members exists, but it is supposed to have a role to protect tissue from the damage by free-radical of oxygen in the acute phase of inflammation.

In Japan, more than 30 applications for patent in the field of lactoferrin and enzyme hydrolysate of lactoferrin have been published. Among these applications, none is found to be relevant to the treatment and prevention of Wilson's disease and heavy-metal poisoning by using lactoferrin or its enzyme hydrolysate. Eight patent applications which are relevant to the treatment and prevention of hepatitis by using lactoferrin as an active ingredient have been detected. However, each of these eight applications can not impair the novelty, inventive step and industrial applicability of the present invention. This is because symptoms or diseases such as Wilson's disease and heavy-metal poisoning affect not only the liver but also the nervous system, circulatory organs and the kidney, and these prior art applications do not disclose the relationship between LF and heavy metals.

Major information about LF and Wilson's disease which forms the background of the present invention is listed as Patent Documents 1 to 8 and Nonpatent Documents 1 to 19.
Patent Document 1: Official gazette of Japanese Provisional Patent Application (Laid-Open) No. 2002-161050 (New Pharmaceutical Composition for Ameliorating Quality of Life, Method for Producing New Food and Use Thereof)
Patent Document 2: Official gazette of Japanese Provisional Patent Application (Laid-Open) No. 2004-359647 (Liposome-Containing Composition for Oral Administration)
Patent Document 3: Official gazette of Japanese Provisional Patent Application (Laid-Open) No. 2002-332242 (Therapeutic Agent for Chronic Viral Hepatitis Type C)
Patent Document 4: Official gazette of Japanese Provisional Patent Application (Laid-Open) No. 2001-247474 (Drug for Preventing and/or Treating Liver Disease)
Patent Document 5: Official gazette of Japanese Provisional Patent Application (Laid-Open) No. 2000-325046 (Food or Drug for Preventing and Treating Hepatitis)
Patent Document 6: Official gazette of Japanese Provisional Patent Application (Laid-Open) No. 2001-517939 (Peptide-Enhanced Transfections)
Patent Document 7: WO02/043753 (Therapeutic Agent for Chronic Hepatitis B)
Patent Document 8: WO02/043752 (Interferon Therapeutic Effect- Potentiating Agents)
Nonpatent Document 1: Hisao Hayashi et al., Yakugaku Zasshi (Pharmaceutical Magazine) 124: 711-724, 2004
Nonpatent Document 2: RE Tanzi et al., Natl Genet 5: 344-350, 1993
Nonpatent Document 3: JM Walshe, Lancet 1: 25-26, 1956
Nonpatent Document 4: JM Walshe, Lancet 2: 1401-1402, 1969
Nonpatent Document 5: JM Walshe, Orphan Drugs, FE Karch, Ed. (Marcel Dekker, New York) pp57-71, 1982
Nonpatent Document 6: GJ Brewer et al., Ann Intern Med. 99(3): 314-9, 1983.
Nonpatent Document 7: GJ Brewer et al., Arch Neurol. 51: 545-554, 1994
Nonpatent Document 8: Y Shiono et al., Am J Gastroenterol. 96: 3147-3151, 2001
Nonpatent Document 9: A Harashima et al., J Trace Elem Exp. 17: 65-73, 2004
Nonpatent Document 10: JH Brock, Biochem Cell Biol. 80: 1-6, 2002
Nonpatent Document 11: Anderson BF et al., Proc Natl Acad Sci USA 84: 1769-1773, 1987
Nonpatent Document 12: Aisen and Harris, "Iron carriers and iron proteins. Vol 5," edited by Loehr T. VCH Publishers, New York. pp.241-351, 1989
Nonpatent Document 13: Moore et al., J Mol Biol. 274:222-236, 1997
Nonpatent Document 14: S Karthikeyan et al., Acta Crystallogr Sect D Biol Crstallogr. 55: 1805-1813, 1999
Nonpatent Document 15: AK Sharma et al., J Mol Biol. 289: 303-317, 1997
Nonpatent Document 16: CA Smith et al., Biochemistry 31: 4527-4533, 1992 Nonpatent Document 17: MO Purina et al., Biochem Cell Biol. 80: 35-39, 2002
Nonpatent Document 18: MC Yoshida et al., J Hered. 78:361-5, 1987
Nonpatent Document 19: T Okayasu et al., Pediatr Res. 31: 253-7, 1992

### DISCLOSURE OF THE INVENTION

The present invention is intended to provide an agent for ameliorating symptoms or diseases caused by heavy metals, such as Wilson's disease, heavy metal intoxication, aging, fulminant hepatitis and so on, which has a high level of safety without any fear of side effects, and which can prevent or decrease the accumulation of heavy metals in the body by eliminating heavy metals such as copper ion accumulated in excess in the body, to eliminate and decrease its effects; and a medicinal composition, a food and a cosmetic containing the same.

In order to accomplish the above-mentioned purpose, the present inventors eagerly searched for a wide range of heavy metal chelating compounds which are included in natural materials, and have found that lactoferrin is a compound which (1) has a high safety and (2) can excrete heavy metal ions like copper ion excessively accumulated in the body into bile, and that it can prevent and treat disorders caused by accumulation of copper in LEC rats, which are model animals of Wilson's disease. Moreover, the present inventors have found that lactoferrin forms complex compounds with heavy metal ions, such as mercury, lead, nickel, chromium, cobalt and the like accumulated in the body, and that lactoferrin can safely excrete them to the outside of the body, to accomplish the present invention.

More specifically, the present invention provides:
(1) an agent for ameliorating symptoms or diseases caused by a heavy metal, comprising lactoferrin and/or an active derivative thereof as an active ingredient;
(2) the ameliorating agent according to the above (1), wherein the symptoms or diseases caused by a heavy metal is one or more of Wilson's disease, heavy metal intoxication, aging, and fulminant hepatitis;
(3) the ameliorating agent according to the above (1) or (2), wherein the heavy metal is selected from the group consisting of iron, copper, mercury, lead, chromium, nickel, manganese and cobalt;
(4) the ameliorating agent according to any of the above (1) to (3), wherein the lactoferrin and/or an active derivative thereof is apolactoferrin and/or a lactoferrin derivative bound to polyoxyethylene glycol;
(5) a medicinal composition comprising the agent for ameliorating symptoms or diseases caused by a heavy metal according to any of the above (1) to (4);
(6) the medicinal composition according to the above (5), wherein the medicinal composition is a preventive or therapeutic agent for Wilson's disease or heavy metal intoxication;
(7) a food comprising the agent for ameliorating symptoms or diseases caused by a heavy metal according to any of the above (1) to (4); and
(8) a cosmetic comprising the agent for ameliorating symptoms or diseases caused by a heavy metal according to any of the above (1) to (4).

The ameliorating agent of the present invention also serves as an agent for preventing or decreasing the accumulation of heavy metals in the body, or an agent for promoting the excretion of heavy metals from the body.

The present invention provides an agent comprising lactoferrin as an active ingredient for ameliorating Wilson's disease, heavy metal intoxication and the like, which has an extremely high level of safety, and compositions such as a medicinal composition, a food and a cosmetic containing the same for prevention and/or treatment of Wilson's disease and heavy metal intoxication (hereinafter may be referred to as the ameliorating agents and the like). The agents conventionally used for the prevention and treatment of Wilson's disease and heavy metal intoxication have been low molecular weight compounds having nature of forming a complex compound with a heavy metal ion, for which side effects upon administration are inevitable. On the contrary, the ameliorating agents and the like of the present invention have an extremely high safety and can ensure improvement of the disease conditions. Further, because LF is a component of milk, mucus or neutrophils, it can be used simultaneously with other agents, without adversely affecting the action of the other agents.

The ameliorating agents and the like of the present invention are useful for the prevention before the onset and the treatment after the onset of symptoms or diseases caused by heavy metals, such as Wilson's disease and heavy metal intoxication. There is no case similar to the excretion using lactoferrin of heavy metals including copper and iron accumulated in the body. Its application is not limited to the prevention and treatment of symptoms or diseases such as Wilson's disease, heavy metal intoxication, fulminant hepatitis and so on. Because it is useful for the prevention and treatment of oxidant stress catalyzed by heavy metal ions, it can also be used for the purpose of prevention of aging (anti-aging) .

### BRIEF DESCRIPTION OF THE DRAWINGS

Fig. 1 shows the survival rates of the LF- and control- groups of LEC rats over time in Experiment 1.
Fig. 2 shows the mechanism of LF (apolactoferrin) that excretes heavy metal ions accumulated in a peripheral tissue and the liver.

### BEST MODE FOR CARRYING OUT THE INVENTION

The ameliorating agents and the like of the present invention are characterized in that lactoferrin and/or an active derivative thereof is comprised as an active ingredient. Lactoferrin used in the present invention is a known substance and is commercially available. In general, lactoferrin is prepared from milk of mammals. The sources include milk of cows, buffalos, humans, pigs, sheep, goats and horses. In order to produce lactoferrin, known methods, for example, the method for purifying lactoferrin using a sulfonated carrier can be advantageously and industrially used. In the present invention, lactoferrin produced by a genetic engineering procedure as well as the one purified from milk of a transgenic animal can also be used. These LFs purified from natural sources and LFs which are substantially equivalent to them are conveniently called "natural-type" LFs.

The bovine lactoferrin extracted from cow milk or lactoserum contains 10-15 % of chelated ferric ions. In order for the treatment and prevention of Wilson's disease, the prevention of aging (anti-aging) and so on, it is not desirable to have iron ions incorporated into the body. Therefore, when using LF with regard to the present invention, it is desirable to use apolactoferrin from which substantially all chelating ferric ions have been completely eliminated.

An active derivative of lactoferrin in the present invention means any alternation of a natural-type LF as long as it has an action for ameliorating at least one symptom or disease caused by a heavy metal. For example, a so-called pegylated lactoferrin, which is LF bound to polyoxyethylene glycol, as well as enteric-coated lactoferrin can also be used. Moreover, fragmented lactoferrin can be used as long as it retains an action for ameliorating a heavy metal disorder. The presence or absence of an action for ameliorating a symptom or disease caused by a heavy metal may be verified by, for example, the method mentioned below and so on.

Any of the above-mentioned lactoferrin may be used with regard to the present invention.

The ameliorating agent of the present invention contains LF as the only essential component. The ameliorating agent of the present invention may be a composition as described below, by comprising other component or components.

The composition of the present invention is a composition that contains, in addition to the agent for ameliorating symptoms or diseases caused by heavy metals of the present invention, one or more components which have no adverse effect on the living body or are physiologically acceptable. The medicinal composition of the present invention containing the agent for ameliorating symptoms or diseases caused by heavy metals refers to a composition that contains one or more medicinally acceptable components in addition to the ameliorating agent of the present invention, for example, a composition that will exhibit ameliorating effects such as the prevention or treatment for Wilson's disease, heavy metal intoxication and the like by systemic administration via injection or oral administration. As a matter of convenience, the medicinal composition is not restricted to the one that is to be applied to humans; it also includes the one which is to be applied to animals (a veterinary drug), particularly to mammals.

With regard to the present invention, among those compositions containing the ameliorating agent of the present invention, foods (foods and drinks) of the present invention containing the ameliorating agent for symptoms or diseases caused by heavy metals refer to compositions for oral administration containing the ameliorating agent of the present invention compounded in nutritional supplements or foods and drinks, either per se or after formulated to powders, granules, tablets, capsules or drinks. Further, with regard to the present invention, among those compositions containing the ameliorating agent of the present invention, compositions for local applications are referred to as cosmetics, as a matter of convenience, regardless of whether they are medicinal compositions or not. Methods for manufacturing these various compositions are known to those skilled in the art.

The ameliorating agents and the like of the present invention exert their effects, for example, via injection or oral administration. When the ameliorating agent of the present invention is orally administered, lactoferrin as an active ingredient can be administered per se, but it can be used after being formulated into powders, granules, tablets, capsules or drinks in accordance with conventional methods. With regard to the present invention, oral agents such as powders, granules, tablets, and capsules and so on are formulated according to conventional methods using excipients such as starch, milk sugar, sucrose, mannitol, carboxymethylcellulose, cornstarch, mineral salts and the like.

In addition to the above mentioned excipients, binding agents, disintegrating agents, surfactants, lubricant agents, fluidity promoting agents, coloring agents, flavoring agents and so on may be appropriately used in this kind of formulations. More specifically, binding agents include, for example, starch, dextrin, gum arabic, gelatine, hydroxypropyl starch, sodium carboxymethyl cellulose, methyl cellulose, crystalline cellulose, ethyl cellulose and polyvinylpyrrolidone. Disintegrating agents include, for example, starch, hydroxypropyl starch, sodium carboxymethyl cellulose, cross-linked sodium carboxymethyl cellulose, crystalline cellulose, carboxymethyl cellulose and so on. Surfactants include soybean lecithin, sucrose fatty acid ester and so on; lubricant agents include talc, wax, sucrose fatty acid ester, hydrogenated vegetable oil and so on; fluidity promoting agents include silicic anhydride, dried aluminum hydroxide, magnesium silicate and so on.

Furthermore, by compounding lactoferrin with components such as D-penicillamine, trientine hydrochloride, TTT and salts of zinc, which have been conventionally considered to have an effective action for Wilson's disease and heavy metal intoxication, even better preventive and therapeutic actions for Wilson's disease and heavy metal intoxication may be expected.

When formulating the medicinal composition or cosmetic containing the ameliorating agent of the present invention, it is possible to incorporate commonly used known components depending on the intended use to prepare various forms such as liquid, solid, semisolid and so on. For example, the medicinal composition or cosmetic of the present invention may be prepared by mixing the ameliorating agent of the present invention with higher fatty acid lower alkyl esters such as stearyl alcohols, isopropyl myristate, and so on; animal oils and fats such as lanolin and so on; polyvalent alcohols such as glycerin; surfactants such as glycerin fatty acid ester, monostearate polyethyleneglycol and so on; inorganic salts, waxes, resins, water; and, if necessary, preservatives such as methyl paraoxybenzoate, butyl paraoxybenzoate, and so on.

The effective amount of the ameliorating agent of the present invention or the medicinal composition, food and the like containing the agent by oral administration is not uniform and may be appropriately determined depending on the form of preparation, method of administration, intended use, and the age, weight and disease conditions of the subject of administration (patient). According to the results of animal experiments using rats, it has been shown that lactoferrin should be administered at 5 or more mg/kg body weight of an LEC rat in order to obtain the improving effect of symptoms or diseases caused by heavy metals or the preventive and therapeutic effects for Wilson's disease.

Therefore, although the effective amount differs depending on the form of agents and the like, based on the total amount of compositions applied, lactoferrin may be preferably included in the range of 1 mg/kg to 50 mg/kg per day. For example, an adult person can fully expect the effects by the lactoferrin intake of 100 to 300 mg per day or more, and thus such a necessary amount may be assured in the compositions. If necessary, the daily amount may be administered in several divided doses.

The present invention is explained in detail in the following examples and experiments. It is noted that these examples and experiments are only exemplification of the embodiments and effects of the present invention, and are not restrictive to the present invention in any sense.

### EXAMPLES

### Preparation of ameliorating agent consisting of apolactoferrin

Apolactoferrin was prepared from a natural-type lactoferrin as the material. Specifically, 100 g of lactoferrin extracted and purified from fresh milk (the degree of saturation of ferric ion: 12 %; and the degree of purity: 93 %) by Tatua Biologics, New Zealand, was dissolved in pure water containing 0.1 % of citric acid (5 liters). The solution was filtrated with an ultrafiltration membrane system installed with a ultrafiltration membrane having the molecular weight cut off of 20,000 dalton, and the retention liquid containing apolactoferrin was washed once with 5 liters of 0.1 % citric acid solution and then three times with 5 liters of pure water. The water was removed from the retention liquid containing apolactoferrin with a reverse osmosis membrane to obtain a concentrated solution, which was then freeze-dried to yield 95 g of white apolactoferrin. The degree of purity of the obtained apolactoferrin was 94 %, and the iron content was a trace level.

### EXPERIMENTS

LEC rats used in the following experiments were discovered from a closed colony of Long-Evans rats and established as model animals that spontaneously develop hepatitis and liver cancer at the Center for Experimental Plants & Animals of Hokkaido University (now Center for Advanced Science and Technology) [Nonpatent Document 18]. However, it has been recently demonstrated that the cause of the diseases of LEC rats is an abnormal accumulation of copper in the liver and nervous associated with a genetic abnormality of copper metabolism. Because the characteristics of this inherited disease coincide well with the cause of Wilson's disease in humans, LEC rats are positioned as the best model animal of Wilson's disease in humans [Nonpatent Document 19].

LEC rats have cinnamon-like colored hair. At around four to five months after birth, about 80 % of LEC rats spontaneously develop acute hepatitis exhibiting severe jaundice and weight loss as main symptoms, as well as anemia, hematuria, oliguria and subcutaneous bleeding. Furthermore, about 50 % of them show severe symptoms with accompanying renal failure, and die within about two weeks from the onset of hepatitis. The mortality rate differs depending on sex, with 70 % for female and 40 % for male. In particularly severe cases, remarkable deposition of subcutaneous bilirubin, bleeding in a nasal part and subcutaneous bleeding are seen, and atrophied liver with red to brown color, slightly enlarged spleen, and yellow kidney are observed after celiotomy, which resembles the clinical and pathological pictures in human fulminant hepatitis. Therefore, LEC rats are also positioned as the model animal of fulminant hepatitis caused by the oxidant stress due to metal ions.

About a half of LEC rats that developed acute hepatitis goes into remission. The incidence rate of hepatitis somewhat varies depending on the growing conditions and differences among individuals, but 10 % of survived rats have a recurrence of hepatitis and die later. About 40 % of all rats survive one year or more, but almost all rats finally develop hepatocellular carcinoma. Therefore, LEC rats are the model animal of liver cancer as well as the one of Wilson's disease.

Twenty female LEC rats of 10 weeks-old were randomly divided into two groups; one was the control group (n=10), and the other was the LF group (n=10). Both groups had unlimited access to water and feeding. The control group was fed on standard powder feed for rats (CLEA Japan, Inc., CE-2), whereas the LF group was fed on the same feed as the control group supplemented with 2 % of lactoferrin bulk powder (LF bulk powder extracted from cow milk, the degree of iron saturation: 12 % and the degree of purity: 93 % ; purchased from Tatua Biologics, New Zealand). Each group was separately grown, and the mortality rates (the survival rates) over time were compared.

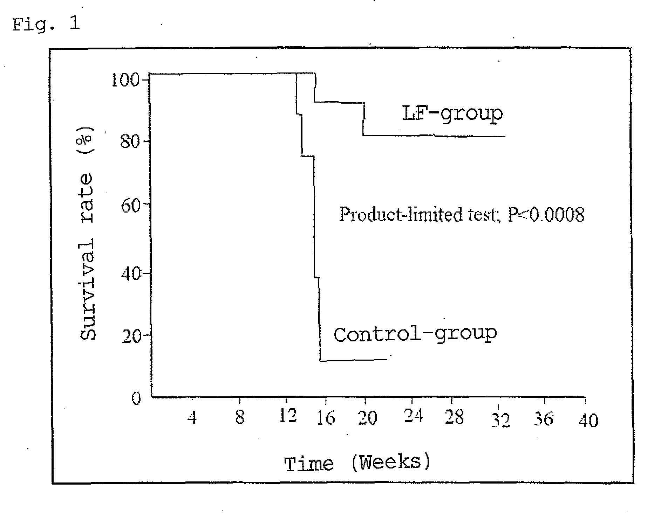

The results are shown in Fig. 1. In the control group, 90 % of the rats died in the 26th to 30th week after birth, developing hyperbilirubinemia followed by jaundice. In contrast, 80 % of the LF group survived even after 35 weeks (P<0.01; according to X²-test). In other words, the survival rates after 36 weeks were 80 % for the LF group whereas 10 % for the control group.

When apolactoferrin which was free of ferric ions was added in a similar experiment, it has been confirmed that apolactoferrin was more effective than a natural-type lactoferrin for the accumulation of copper in LEC rats. When a natural-type lactoferrin with the degree of iron saturation of 12 to 15 % was compared with ferric ion-free apolactoferrin, the difference in the effectiveness may not be statistically significant, but it is considered that apolactoferrin is more suitable for the ameliorating agents and the like because it does not bring ferric ions into the body.

The above experiments have revealed that lactoferrin can prevent disorders of the liver, kidney, nervous system and so on of LEC rats due to the accumulation of copper. Considering that LEC rats serve as a pathological animal model which closely resembles the pathology of Wilson's disease as well as a pathological animal model of fulminant hepatitis and liver cancer caused by metals, it is evident that lactoferrin is effective as a preventive and/or therapeutic agent having a high level of safety for symptoms or diseases caused by metals (particularly heavy metals) such as Wilson's disease, fulminant hepatitis, liver cancer and so on.

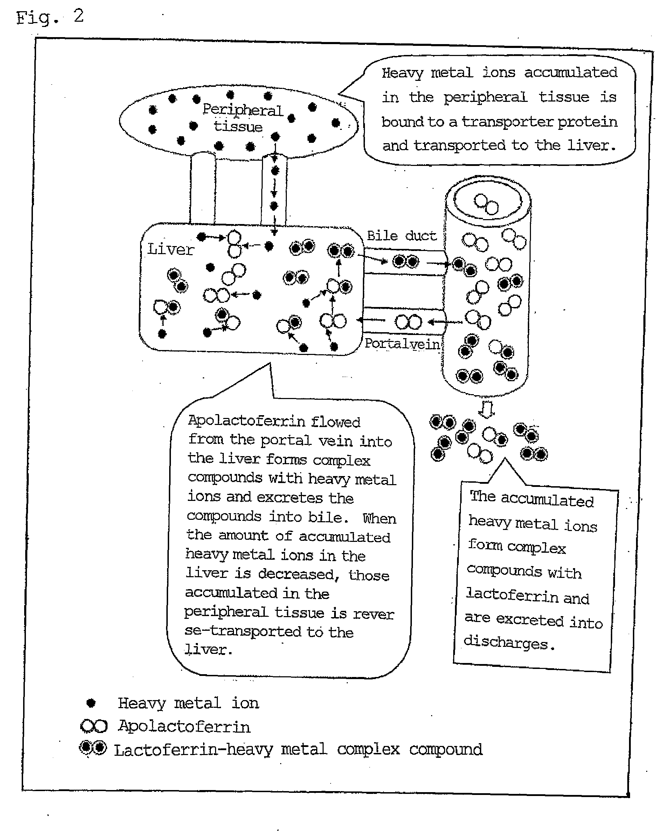

Moreover, the fact that even a natural-type lactoferrin is fully effective suggests a mechanism in which lactoferrin is absorbed from the small intestine as a whole molecule, despite it is a high molecule having a molecular weight exceeding 80,000 daltons, arrives at the liver, forms complex compounds with accumulated copper ions, and excretes the compounds into bile. Fig. 2 shows the mechanism that lactoferrin excretes heavy metal ions accumulated in a peripheral tissue and the liver. Apolactoferrin absorbed from the jejunum and the intestinum ileum forms complex compounds with heavy metal ions in the liver, which are dissolved in bile and then excreted from the bile duct into the small intestine. The complex compound of the excreted lactoferrin and heavy metal ions passes through the colon and is excreted with discharges to the outside of the body.

In addition, it has been cleared that lactoferrin may prevent and treat Wilson's disease and heavy-metal poisoning by forming complex compound with heavy-metal ion accumulated in the body with aging and excreting the compound to the outside of the body.

The present application is based on Japanese Patent Application No. 2005-189619 filed on June 29, 2005. The content described in the specification and claims of Japanese Patent Application No. 2005-189619 are all incorporated here in the specification of the present application.

## Claims

1. An agent for ameliorating symptoms or diseases caused by a heavy metal, comprising lactoferrin and/or an active derivative thereof as an active ingredient.

2. The ameliorating agent according to claim 1, wherein the symptoms or diseases caused by a heavy metal is one or more of Wilson's disease, heavy metal intoxication, aging, and fulminant hepatitis.

3. The ameliorating agent according to claim 1 or 2, wherein the heavy metal is selected from the group consisting of iron, copper, mercury, lead, chromium, nickel, manganese and cobalt.

4. The ameliorating agent according to any of claims 1 to 3, wherein the lactoferrin and/or an active derivative thereof is apolactoferrin and/or a lactoferrin derivative bound to polyoxyethylene glycol.

5. A medicinal composition comprising the agent for ameliorating symptoms or diseases caused by a heavy metal according to any of claims 1 to 4.

6. The medicinal composition according to claim 5, wherein the medicinal composition is a preventive or therapeutic agent for Wilson's disease or heavy metal intoxication.

7. A food comprising the agent for ameliorating symptoms or diseases caused by a heavy metal according to any of claims 1 to 4.

8. A cosmetic comprising the agent for ameliorating symptoms or diseases caused by a heavy metal according to any of claim 1 to 4.
